# EUROPEAN PATENT SPECIFICATION

(11) **EP 1 457 913 B1**
(45) Date of publication and mention of the grant of the patent: **17.12.2008**
(21) Application number: 04014204.4
(22) Date of filing: 30.07.1999
(51) Int. Cl.: A61B 5/00, G06F 19/00

(54) **Method of managing data for a plurality of analyte test instruments**
Verfahren zur Datenverwaltung für eine vielzahl von Analyttestvorrichtungen
Procédé pour traiter les données d'un grand nombre d'instruments d'analyse

(30) Priority: 31.07.1998 US 94895 P
(43) Date of publication of application: 15.09.2004
(62) Divisional of application: 99938895.2
(73) Proprietor: ABBOTT LABORATORIES, Abbott Park IL 60064-3500 (US)
(72) Inventor: Krishnaswamy, Sarath, Chelmsford, MA 01824 (US); Guiney, Patrick, Concord, MA 01742 (US)
(74) Representative: Modiano, Micaela Nadia

(56) References cited:
- EP-A- 0 251 520
- EP-A- 0 387 630
- EP-A- 0 684 575
- WO-A-94/11838
- WO-A-94/24929

## Description

### BACKGROUND OF THE INVENTION

Health care professionals at medical institutions are routinely required to use various instruments to perform bedside tests on patients to monitor various aspects of patients' health. These tests generate substantial amounts of medical data which is often collected and organized for subsequent analysis. The data can include results of tests to determine the level of one or more analytes (e.g., blood glucose, ketones).

Traditionally, the primary means for collecting and organizing data obtained from the instruments is a printed or transcribed record of the test results. To review the results, a health care professional either retrieves the results from the institution's records department or goes to the patient's room. Since these results are often available only in printed form, chronological and statistical analysis is difficult.

Government regulations require medical institutions to perform control tests on instruments used for patient testing at regular intervals to ensure the accuracy of test results. Health care professionals that operate such instruments are also required to undergo periodic recertification.

Members of the institution's administrative staff are frequently responsible for the review of instrument control test data and recertification procedures to ensure compliance with federal regulations. In many instances, however, administrators identify tests involving "out-of-specification" instruments, expired supplies (e.g., test strips), or uncertified health care professionals after testing has been completed. These test results are either accepted or the patient can be subjected to another test.

It is therefore desirable to have a health data management system in which each of a plurality of medical test instruments are connected to a data communications network to provide real time transfer of patient test results to a centralized location. In addition, it is desirable to include in such a system a security mechanism for preventing testing of patients with "out-of-specification" instruments, expired supplies (e.g., test strips), or uncertified health care professionals.

WO-A-94/11838 discloses a network database system that tracks biological fluids by their identifier through the system to a plurality of testing equipment coupled to the network. The testing equipment includes e.g, incubators and piepettors, with respective monitors connected thereto.

WO-A-94/24329 discloses a patient monitor and support system including a plurality of patient sites individually connected via a set of communications links to a care centre including a plurality of computer workstations. Each patient site is controlled by a base unit in communication with the care centre. Portable recorders, that may record inputs from sensors such as reagent strips, may be docked in docking ports of the base unit to upload or download data and to charge the batteries of the recorders. Said portable recorders have displays.

### SUMMARY OF THE INVENTION

A method according to claim 1 is provided.

### BRIEF DESCRIPTION OF THE DRAWINGS

The foregoing and other objects, features and advantages of the invention will become apparent from the following more particular description of preferred embodiments of the invention, as illustrated in the accompanying drawings. The drawings are not necessarily to scale, emphasis instead being placed on illustrating the principles of the present invention.
FIG. 1 is a perspective view of an analyte test instrument disposed in a docking station.
FIG. 2 is a functional block diagram of a plurality of medical test instruments connected to a host computer over a data communications network.
FIGS. 3A - 3C are top, side and end views, respectively, of an analyte test instrument.
FIG. 4 is a cut-away perspective view of an analyte test instrument.
FIG. 5 is a sample of displayed data provided on a LCD module of an analyte test instrument.
FIG. 6 is a perspective view of a three-electrode test strip for use with an analyte test instrument.
FIGS. 7 is a perspective view of the back of an analyte test instrument opened to expose a battery compartment and a separate rechargeable battery pack.
FIGS. 8A - 8B are illustrations of a rechargeable battery pack and a two finger leaf spring contact connector for use with the rechargeable battery pack, respectively.
FIGS. 9A - 9B are cross-sectional views of an analyte test instrument shown with alkaline power cells and with a rechargeable battery pack, respectively.
FIG. 10 is an illustration of a battery monitoring circuit and recharge current circuit employing a pair of two finger leaf spring contact connectors.
FIGS. 11A - 11B is a perspective view of a docking station for use with an analyte test instrument.
FIG. 12 is a functional block diagram of a docking station switching circuit for directing data transfer between two ports.
FIG. 13 is an illustration of a computer interface cable for use with a docking station.
FIG. 14 is a functional block diagram of a data management system for use with multiple analyte test instruments in a hospital environment.

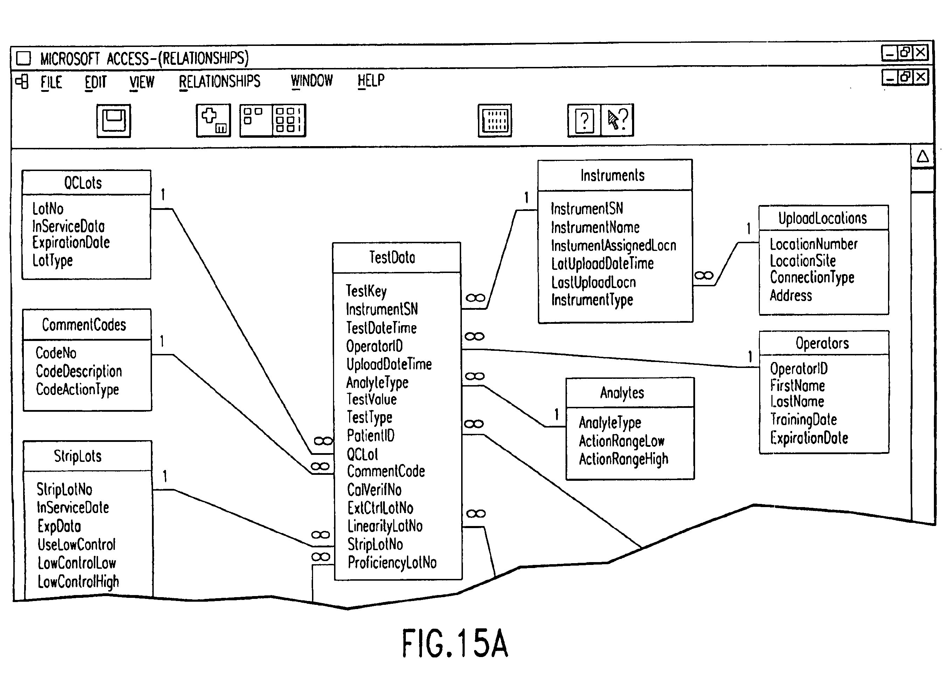

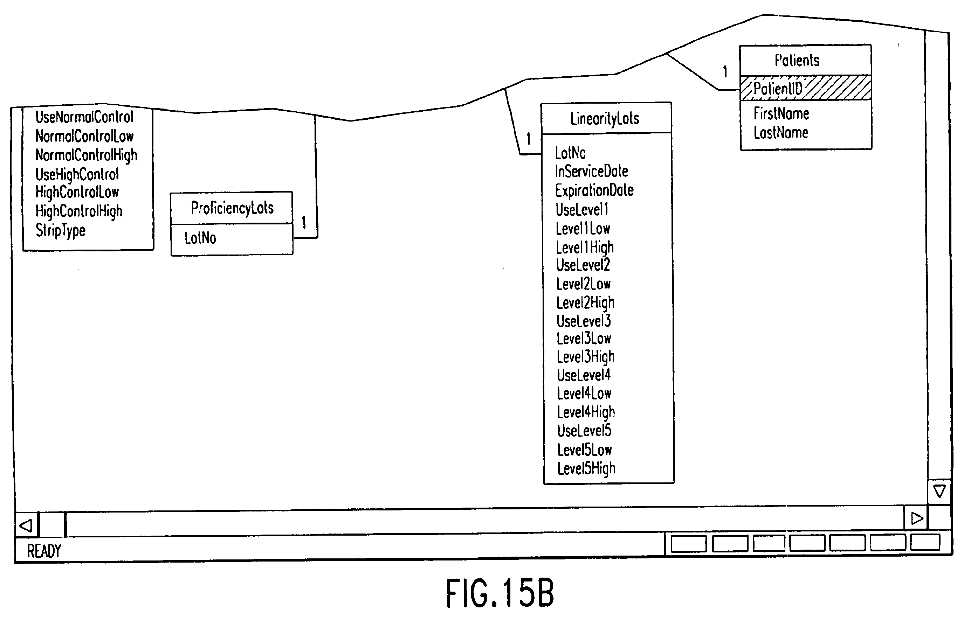

FIG. 15 shows one possible configuration of database tables for use in an analyte instrument data management system.

### DETAILED DESCRIPTION

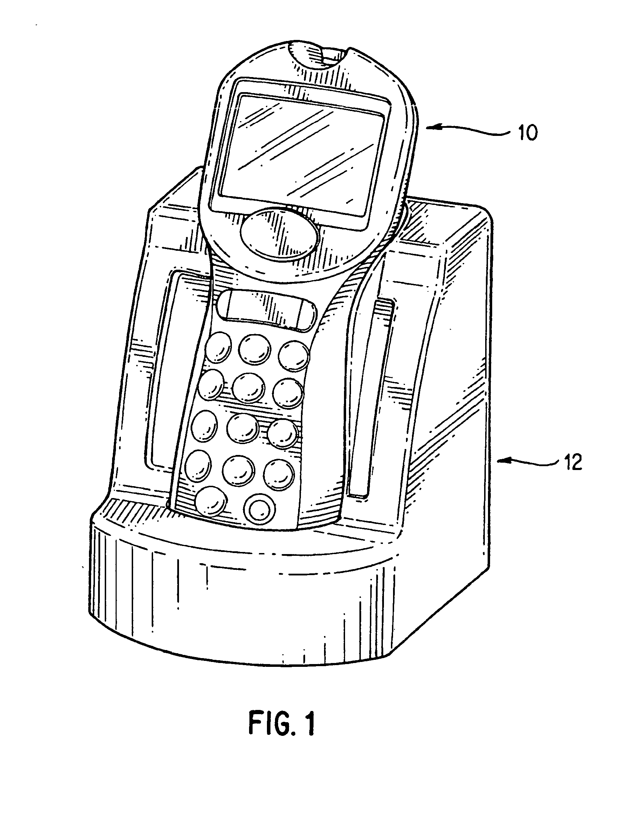

Referring to FIG. 1, an instrument 10 used in patient testing for one or more analytes (e.g., blood glucose, ketones, etc.) in a hospital environment is shown positioned in a docking station 12. The instrument 10 analyzes a patient sample (e.g., blood) deposited on one end of a test strip when the other end of the strip is inserted in the instrument 10. The docking station 12 allows for automatic transfer of test results to a host computer and provides power to recharge an internal battery pack when the instrument 10 is positioned in the station 12.

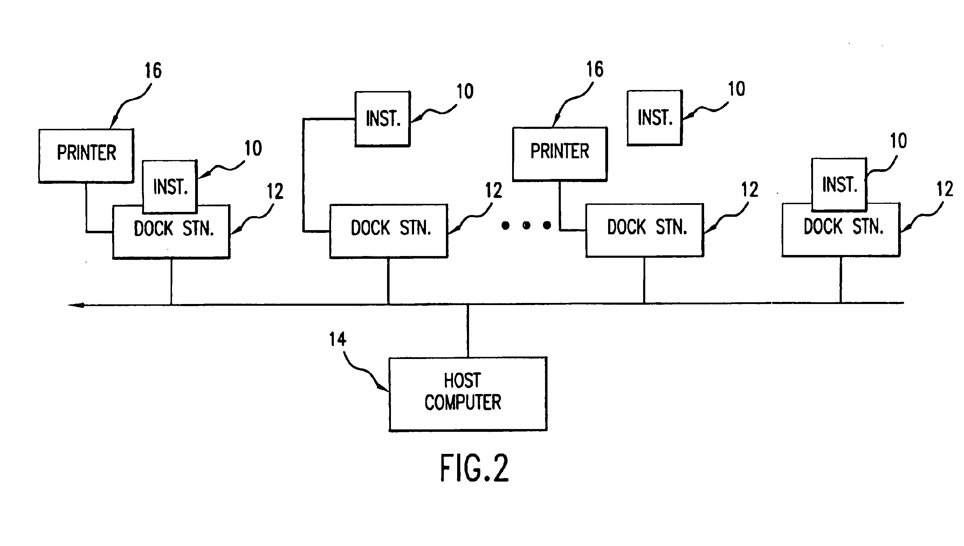

A plurality of instruments 10 are networked to a host computer 14 though docking stations 12 as shown in FIG. 2. For example, one instrument can be assigned to each patient room. A nurse or other operator inserts a test strip into the instrument and deposits a patient sample onto an exposed portion of the test strip. An audible indicator alerts the operator when a sufficient patient sample volume for analysis has been deposited on the test strip. The instrument then analyzes the sample and displays the results on the LCD module. The operator can return the instrument to the docking station 12, even before the results are available, where the test data (operator ID, patient ID, date, time, and other parameters) are automatically transferred via a cable to the host computer 14. The test data and results can be directed to a local printer 16 by a cable (e.g., an RS-232 standard interface cable) to generate a hardcopy, if desired. The network is controlled by the host computer 14 via a bi-directional data communication link, i.e., data can be transferred from the instrument 10 to the computer 14 and data can be transferred from the computer 14 to the instrument 10. The latter mode allows for remote independent configuration of individual instruments or groups of instruments.

### Analyte Test Instrument

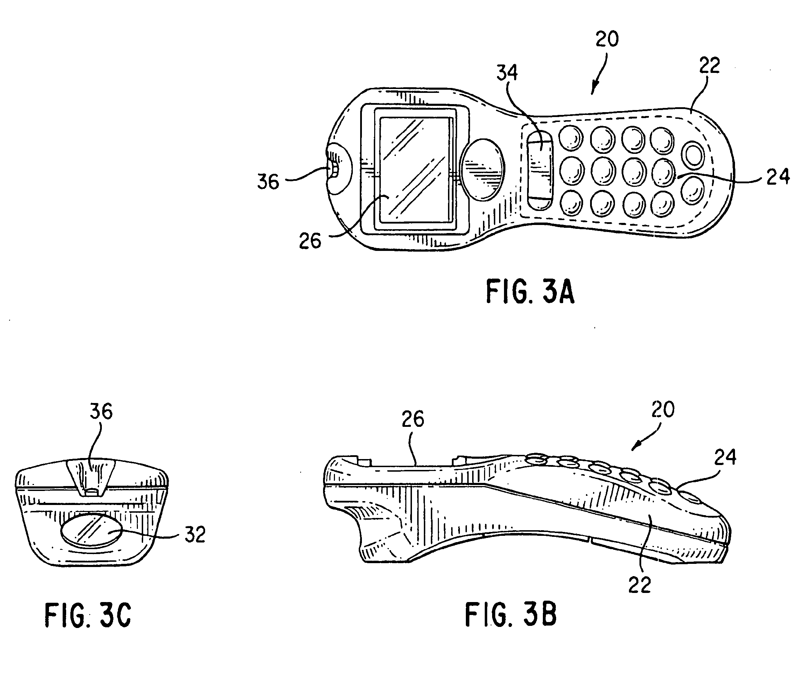

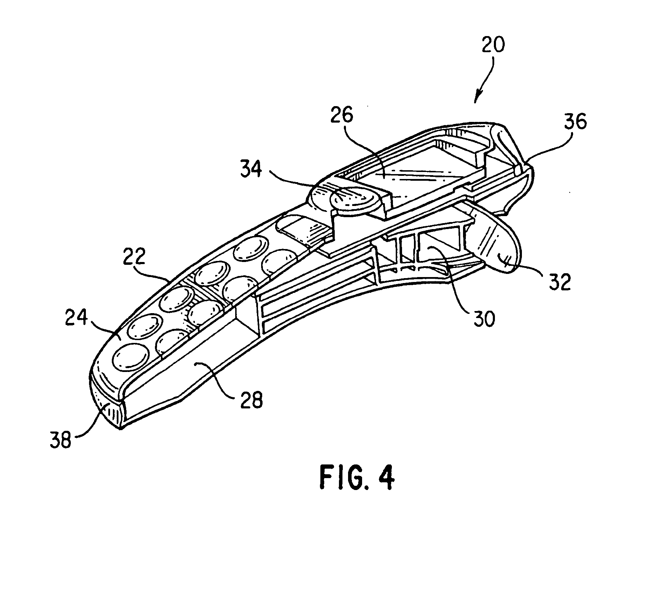

Referring to FIGS. 3 and 4, an analyte test instrument 20 includes a housing 22, a user interface 24, and a display area 26. The housing 22 is shaped to permit hand-held operation for bedside patient testing. The housing 22 includes an internal subframe 28 for mounting an analog and digital printed circuit boards and a barcode scan engine 30. The subframe also forms the battery cavity and includes battery contacts (not shown). The housing 22 is fabricated from rubber or plastic (e.g., ABS Polycarbonate). Smooth surfaces and a minimum of exposed fasteners and seams help to minimize areas which can collect foreign material and facilitates cleaning of the instrument. Silicone rubber pads are attached with adhesive to the bottom of the housing 22 to prevent skidding.

The user interface 24 includes a numeric keypad and function buttons to activate/deactivate power, select test or menu modes, edit entries, terminate entries, and activate a barcode reader as a substitute for manual numeric entry. All buttons in the user interface are fully sealed (e.g., using membrane switches). The keypad and barcode reader allow operators to enter a variety of data, including operator and patient identification (ID) numbers, strip control lot numbers, calibration codes, and to set other instrument parameters (e.g., date time, security intervals, display backlighting). The barcode reader is used for entry of test strip calibration data because it eliminates the need to visually verify a test strip code during each test.

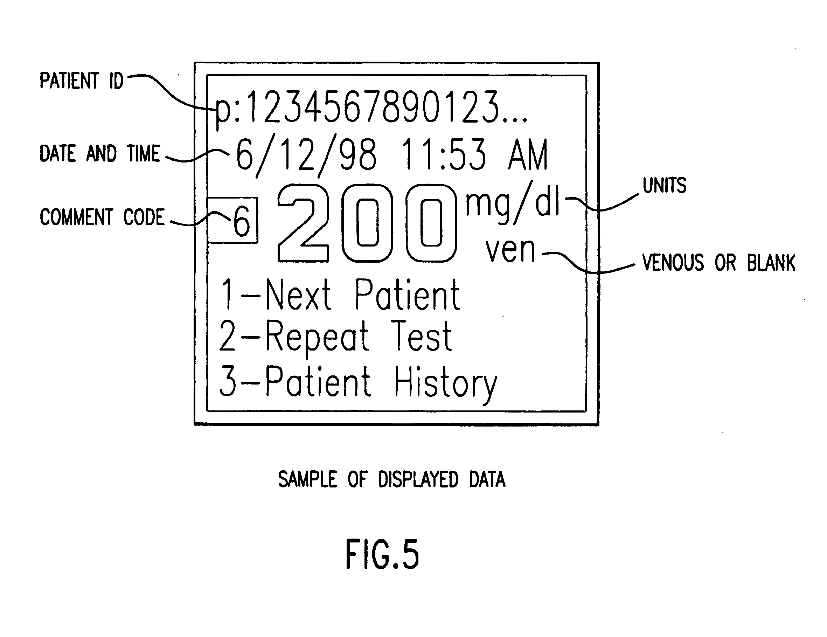

The display 26 is a graphic style liquid crystal display (LCD) module and provides multiple lines of text characters. Referring to FIG. 5, a variety of prompts, audio and visual warnings, and menu items can be displayed along with numerical test results. The display 26 includes a selectable backlight mode utilizing four amber high intensity LED's to improve visibility in poor lighting conditions.

Referring back to FIGS. 3 and 4, the barcode reader comprises a laser scan engine 30 and a red acrylic exit window 32. The red exit window 32 acts as an optical filter to reduce the received light that is not matched to the wavelength of the scan engine laser source (e.g., 680 nm). The barcode reader includes optics disposed at the top of the hand-held instrument to provide non-contact reading of barcodes. The reader is activated by depressing the scan key 34 located at the top of the keypad. The barcode reader can only be activated if the operator is prompted for entry of any one of the following: operator ID, patient ID, or strip lot or control vial information. Identification can be entered manually or read into the instrument via the reader from barcoded identification tags (e.g., wristbands) worn by operators and patients. Barcoded items placed within several inches of the exit window 32 can be scanned after depressing the scan button 34 in the user interface 24. An audible signal indicates successful reading of the barcode.

Barcode readers are well-known in the art (e.g., retail checkout scanners) and are commercially sold by Symbol Technologies, Inc. U.S. Patent No. 5,637,856, which is incorporated herein by reference, describes barcode scanning systems suitable for integration into an analyte instrument.

The instrument 20 includes a test strip port 36 which accepts test strips for determining the level of analyte in a sample taken from the patient. U.S. Patent No. 5,628,890, which is incorporated herein by reference, shows one type of test strip.

A data port ten pin connector 38 is provided in the base of the instrument to allow connection with mating contacts in the docking station for data transfer, battery recharge (from external power source), and printer communication. The connector does not extend beyond the contour of the base end of the instrument. A single row of electrical contacts within the connector is recessed to prevent inadvertent contact with external conductors. The instrument 20 responds to commands uploaded from the host computer linked through the data port. The external computer system initiates data transfer without any action on the part of the operator after the instrument has been mated to the docking station.

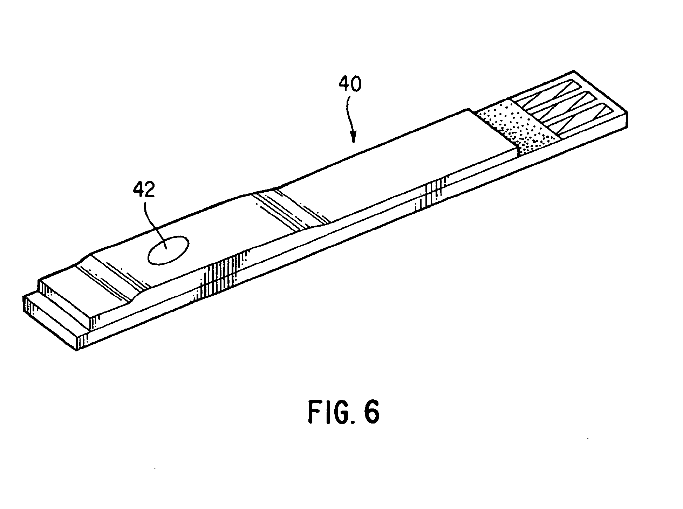

FIG. 6 shows one type of test strip 40 which includes three electrodes and can be used with the instrument (see FIG. 4) for determining the level of an analyte in the blood. The strip is partially inserted into the port 36 so that the sample area 42 remains outside the housing 22. The blood sample is applied to the sample area 42 and flows to an active area (not shown) at the unexposed ends of the three electrodes. The active area creates an electrochemical reaction in the sample, which is monitored electrically. Because each test strip typically has an expiration date, a strip identifier code which can be located on the strip package is either manually entered or scanned by the barcode reader into the instrument 20. If the strip code is not recognized as a valid code, then the instrument 20 alerts the operator and prevents further operation of the instrument 20 with that strip 40.

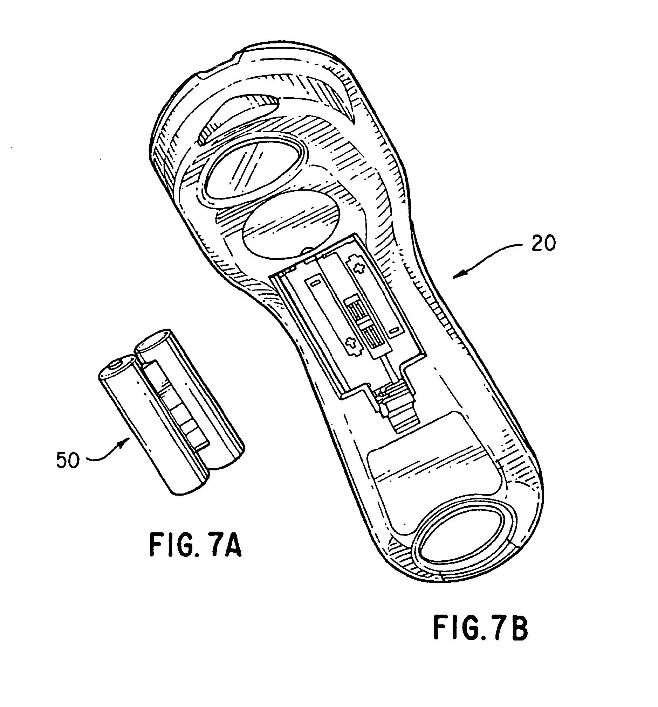

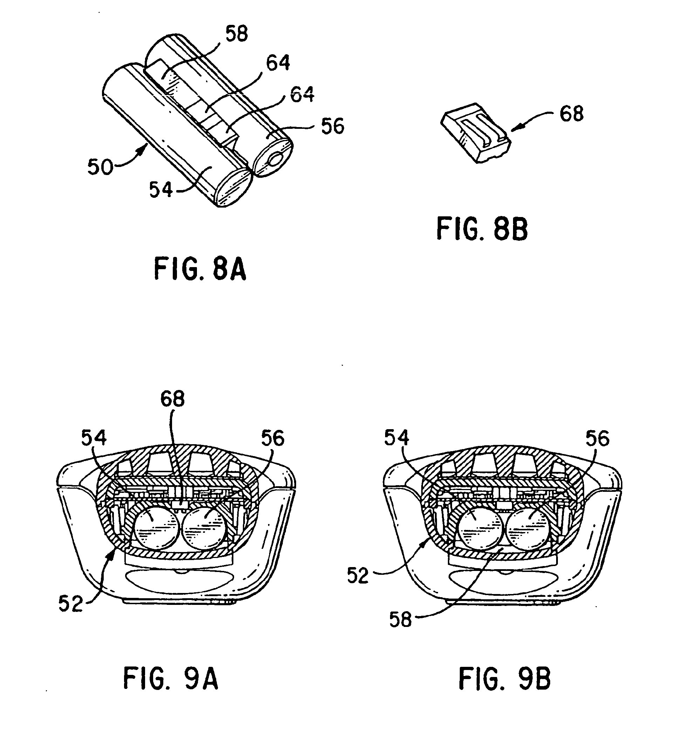

Referring to FIGS. 7 and 8A, the instrument 20 is powered by a rechargeable battery pack 50 (e.g., a nickel metal hydride (NiMH) battery package) securely disposed in a cavity 52 (i.e., battery compartment) on the underside of the instrument 20. The installed battery pack 50 (see FIG. 9A) is recharged by the docking station when the instrument is positioned in the docking station. Alternatively, the instrument can be powered by two standard alkaline batteries which are securely disposed in the same cavity 52 (see FIG. 9A). If an alkaline battery is installed improperly, it will not make electrical contact and the instrument will not turn on.

In addition, the possibility of inadvertently recharging the alkaline batteries is eliminated through the use of the custom-designed rechargeable battery pack 50. Also, keying features in the battery compartment are designed to prevent incorrect insertion of the battery pack or the insertion of a non-specified battery pack, thus eliminating the possibility of another battery chemistry from inadvertently being used.

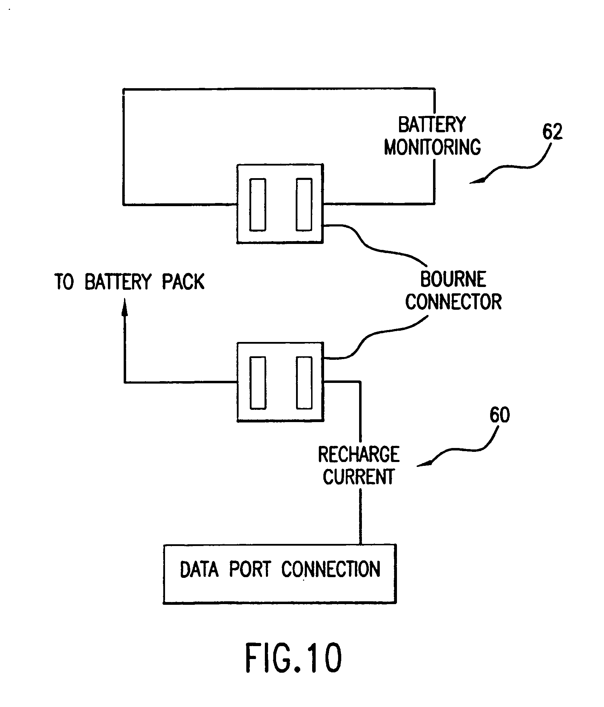

Referring to FIGS. 9A and 9B, the custom-designed battery pack 50 takes advantage of the void space that exists between two standard alkaline batteries 54, 56 when installed in the battery compartment 52, thereby eliminating the possibility of standard alkaline cells from activating the recharge functions. The recharge circuitry includes two independent circuits (see FIG. 10). The first circuit 60 provides recharge current to the rechargeable battery pack 50. The second circuit 62 determines the presence of the rechargeable battery pack 50 in order to facilitate the measuring of battery level. The pack 50 includes a plastic spine 58 which acts as a holder for the two NiMH batteries 54, 56 and occupies the void space which normally exists between two installed alkaline batteries. Referring back to FIG. 8A, two discrete conductive pads 64, 66 located in the plastic spine 58 act as bus bar contacts. Each bus bar contact is used in conjunction with a small two finger leaf spring contact connector 68 (e.g., Bourne connector) located within the void space in the battery compartment 52 (see FIGS. 8B and 9A - 9B). Each finger is electrically independent of the other finger in the connector. When the battery pack 50 is installed, the two electrically discrete bus bar contacts in the plastic spine 58 create an electrical short across each of the two connectors, thereby completing two independent circuits (see FIG. 10). Because completion of the electrical paths requires electrical current to flow from one contact on the connector, through the bus bar contact, and out the other contact of the same connector, there is no possibility that recharge current can be supplied in any other battery system which does not utilize this battery pack configuration.

### Docking Station

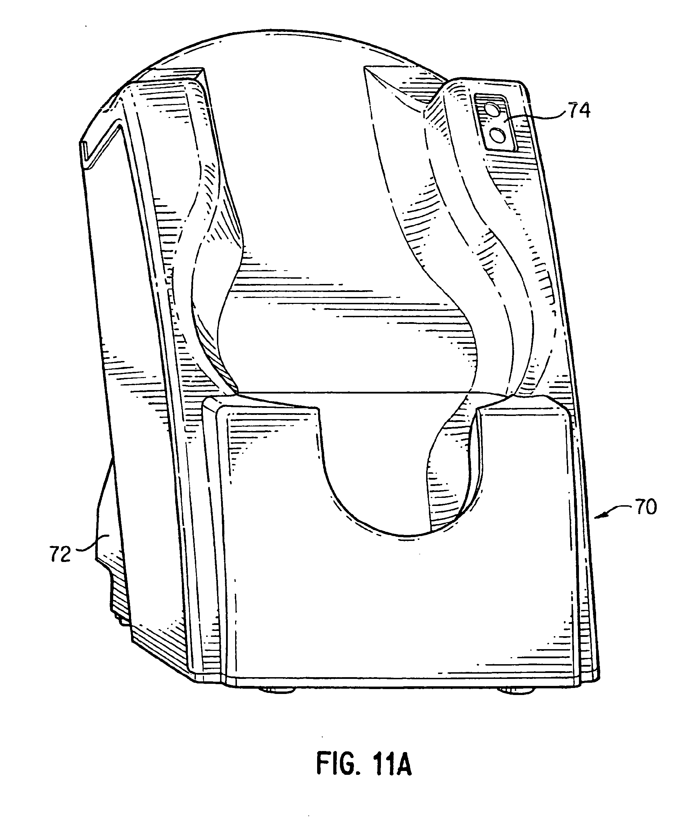

FIG. 11A shows a docking station 70 in a desk mount configuration. An alternate wall mount configuration is achieved by repositioning an attached mounting bracket 72. The docking station 70 provides at least the following two important capabilities for the instrument. First, an instrument with a rechargeable battery pack is recharged when seated in the docking station. Second, data communication with the host computer or other devices can be established through the docking station. In particular, the docking station is capable of hands-free and near real-time transfer of (1) test data to a host computer and (2) configuration data from the host computer. The docking station 70 also serves as a convenient resting place for the instrument when not in use.

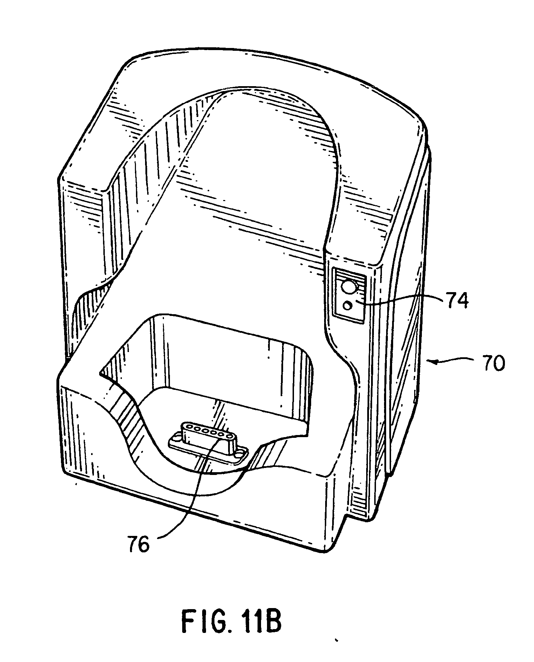

Power is provided to the docking station through an external AC adapter. Status lights 74 (e.g., LEDs) on the docking station indicate when power is on, when a meter has been docked successfully, and when data are being transferred through the docking station. The station 70 includes a docking connector 76 (see FIG. 11 B) which includes a series of electrical contacts and is located in the recessed base. When the instrument is docked, the docking connector 76 receives a low insertion force (LIF) mated connector located in the base of the instrument. One of the station connector contacts provides power to the instrument for recharging the optional battery pack. The battery charge provided to the instrument is a low current (i.e., trickle charge) received from the docking station 70 through the instrument's data port connector 38. The docking station 70 incorporates circuitry to limit overcharging. Although the charging current is available at all times, only instruments equipped with a rechargeable battery pack are capable of receiving this current.

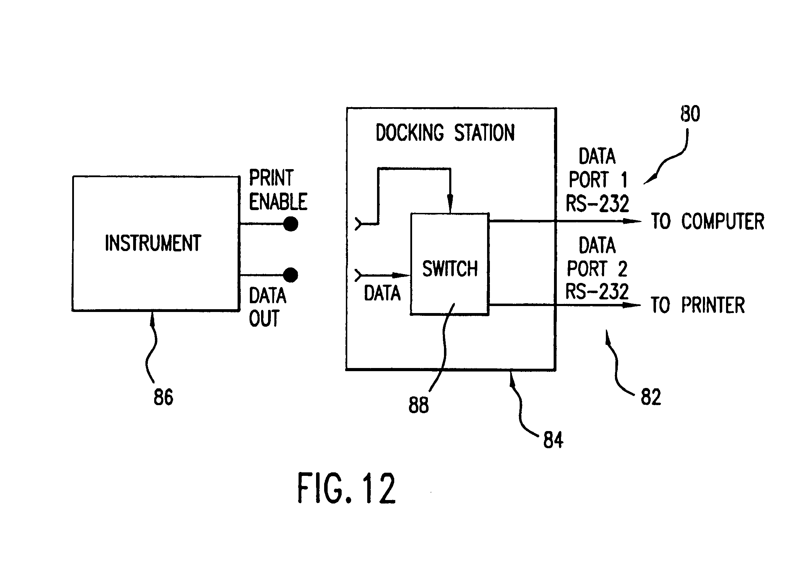

Referring to FIG. 12, the data connection through the docking station is essentially a pass-through connection from the instrument data port connector (see FIG. 4) to one of two standard 9-pin RS-232 ports. A first data port 80 is used for data transfer (e.g., to a computer, a modem, or an Ethernet terminal server) and the other port 82 is available for connection to a peripheral device (e.g., a printer). In its default condition, the docking station 84 is configured to pass data between the instrument 86 and the first data port 80. Data is passed to the second data port 82 when the docked instrument sets a switch 88 for the print mode. After data transfer through the second port 82 is completed, the docking station 84 resets switches to connect back to the first port 80.

The docking station 84 can be connected via a computer interface cable to a computer, a modem serial port, or some other communications port (e.g., Lantronix box) for data transfer over a communication line (e.g., a telephone or Ethernet TCP/IP line). The cable includes a standard nine pin RS-232 connector which mates with the docking station 84. A similar cable is used to communicate with a printer or other external device.

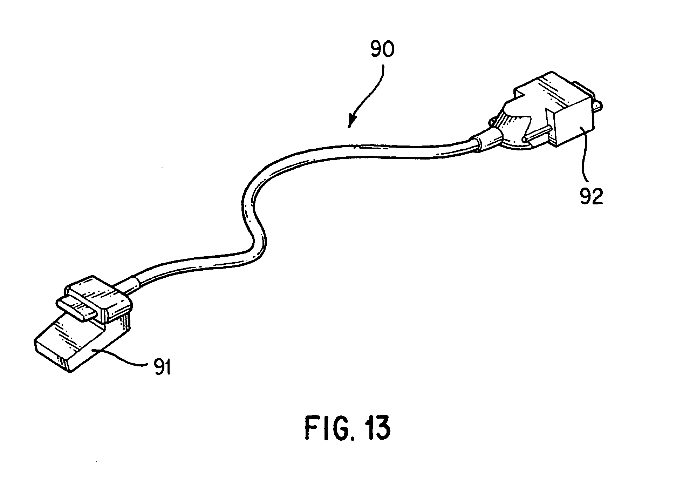

FIG. 13 shows a different computer interface 90 cable which can be used in place of the docking station for direct communication with a computer (e.g., a laptop PC). The cable includes a standard DB9 connector 91 at one end and a RS-232 connector at the other end 92. This cable, however, does not include a means to recharge the battery pack.

### Data Management System

A data management system facilitates the data communication and control between multiple instruments and a computer. The system is particularly advantageous to instruments used in a health care environment. The system allows test data to be automatically uploaded from each instrument to the host computer and subsequent reviewing, graphing and printing of the data. Uploaded data can be made available to other external systems through a specified port (i.e., a data forward port) for use in third party applications. In addition, instrument configuration and security data can be downloaded to the individual instruments according to specific procedures or preferences.

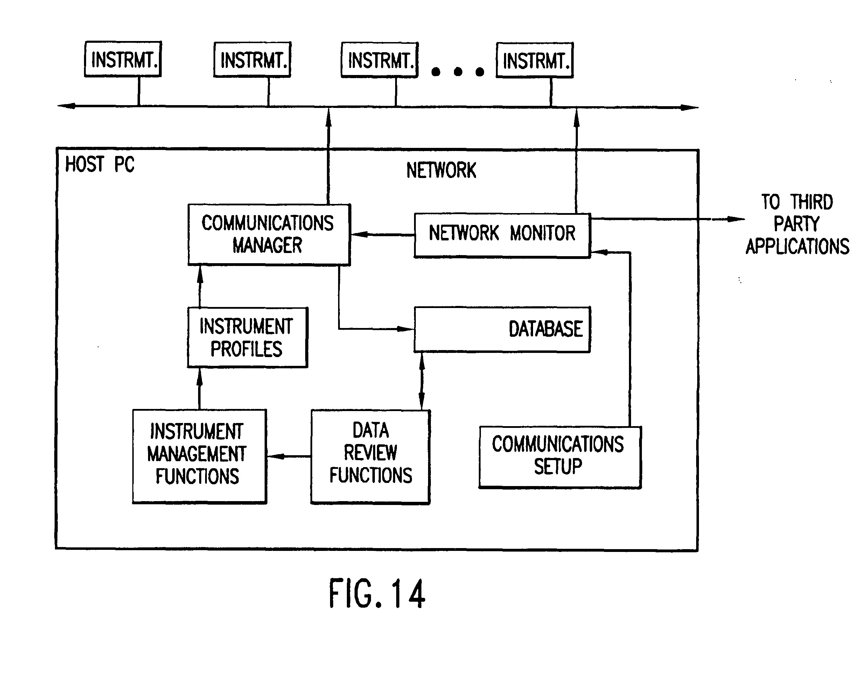

Referring to FIG. 14, a data management (DM) system is shown as a related group of functional blocks. When an instrument is placed in a docking station after testing, the instrument generates a message (i.e., signal) on the network indicating its presence. The host software monitors the network for messages transmitted from the instruments. When a message is received, the host acknowledges the message, determines the location of the docking station, and identifies the particular instrument. The host then reviews its database for instructions for that instrument and sends a set of instrument-specific data (e.g., commands to facilitate data transfer, calibration data) to the instrument before terminating the session. The specific data transferred are determined by the operator of the host computer in a previously executed setup operation. Data from the instruments are stored in a central database which is designed to be accessed by both the DM system and third party users (e.g., independent data applications).

Operators can interact with the DM system to configure upload and download procedures for transferring data to/from specific instruments or instrument groups. Operators can also use the DM system to review test data uploaded from instruments and stored in the database. In addition, operators can remotely monitor instruments and operator performance.

The network monitor function is a background process in the host software that monitors ports on the host computer to detect communication signals from the instruments. The network monitor can check selected TCP/IP ports, modem instruments, and computer serial ports. Once an instrument signal is detected, the network monitor promptly returns an acknowledgment signal to the instrument and determines its identification (i.e., serial number) and location. The network monitor forwards this information to the communications manager and then returns to monitoring the network for communications from other instruments. The network monitoring process can be initiated at the operator's option whenever (1) the host computer is booted, (2) the data review and instrument setup functions are started, or (3) the user specifically starts the network monitor executable. Once started, the network monitor runs continuously on the host unless specifically terminated by the operator. The operator can view the status of all instruments known to the DM system on a summary display screen that is continuously updated as instruments check in.

The communications manager is a set of functions within the host software for controlling data transfer between the host computer and the individual instruments. These functions allow connection to instruments in remote locations and facilitate automatic data transfer (i.e., without human intervention) to and from the instruments at all times. The communications manager opens a communications channel to the instrument and uploads information to the appropriate location in the database. It also downloads previously configured security and setup information to the instrument. Any or all of these functions are specified in advance by the operator using one or more of the instrument management functions. In the event that multiple instruments check in to the network simultaneously, multiple communications manager processes (i.e., one per instrument) can be implemented. Alternatively, a queue of instruments and corresponding network addresses can be established.

Instrument profiles are sets of commands for a group of instruments that are executed when the host computer establishes a connection to an instrument in that group. These commands are used to set the instrument configuration and security options (e.g., date, time, strip lot list, operator list). If there are no instructions for a given instrument, a default profile is used. Profiles are created by the instrument communications library functions which translate commands in the profiles according to the specific instrument type currently connected to the network.

An operator can use data review functions to access the database to view (numerically or graphically) or edit information. In some cases, these functions include data editing capabilities used for entering new data, including lists of operators or new quality control ranges, into the database. These functions also provide notifications or warnings based on a review of data uploaded from the instruments. Notifications can require a user response or acknowledgment for the item that triggered the warning. Warning items can include expired test strip lots, expired QC lots, unqualified operator or any other significant condition. Because these functions include modification of the main database, security procedures (e.g., password protection) are employed to prevent unauthorized modifications. Preferably, any modifications to the database are logged by independent software in an independent log. file. Data review functions also permit a broad range of report generation and manipulation. Reports can include data listings, graphs and statistical information. File management functions allow the user to save, print, or otherwise manage the data files.

The instrument management functions are used to configure data to be sent to an instrument in the hospital. A point-and-click graphical user interface is used to select parameter setting for instrument upload and download, and to create data lists to be downloaded. The user interface includes instrument-specific dialogs that allow the user to configure setup items (i.e., parameters that affect instrument performance that are not directly related to the test). For example, the user interface includes a means for the user to review a list of operators in the database and to select a subset of these operators to download to an instrument. Similarly, a list of acceptable strip lots can be downloaded to each instrument. The download data are in the form of an instrument profile which can be activated at a later time when the instrument next connects in to the network (i.e., is returned to a docking station). An instrument grouping utility allows the user to create, modify and name groups of instruments within the hospital. All instruments within a given group share the same profile. Instrument setup functions are used to establish instrument settings and how the instrument performs its tests. Instrument security functions utilize operator and test strip lot lists stored in the database to establish which operators or test strips can be used with a given instrument.

The database used in the DM system is a standard commercially-available database (e.g., Access™, Oracle™) to allow access by other systems or devices. One possible configuration of database tables is shown in FIG. 14. The database stores test records from each device and can include parameters such as analyte type (e.g., glucose, ketones), test type (e.g., patient, control, etc.), operator ID (i.e., name, training date and/or expiration date), time and date of test, time and date of upload, strip lot data (e.g., QC ranges, service and/or expiration dates), patient ID, control lot ID, instrument name and assigned location, location of upload, pass/fail indication, and comment codes (including text descriptions of numeric comment codes).

## Claims

1. A method of managing data for a plurality of hand-held analyte test instruments (10,86) removably received in respective docking stations (12,70,84) and connected to a data communication network comprising the steps of:
reading calibration data associated with a test strip (40) with a barcode reader;
inserting the test strip (40) into a port (36) of a first hand-held test instrument of the plurality of hand-held analyte test instruments (10,86);
applying a sample to the test strip (40);
processing an analyte signal received from the test strip (40) to generate analyte data;
connecting the first hand-held test instrument to one of the docking stations (12,70,84);
detecting via a host computer (14) the connection of each instrument (10,86) to the data communication network;
uploading data received from each instrument (10,86) to the host computer (14) including the analyte data;
processing the uploaded data on the host computer for operator review;
downloading configuration data from the host computer (14) to each test instrument (10,86), the downloaded data comprising instrument specific set up and control data.

2. A method according to claim 1 wherein each of the analyte test instruments (10,86) includes a test strip port (36), which accepts test strips (40) for determining the level of analyte in a sample taken from a patient.

3. A method according to claims 1 or 2 further comprising a security system for preventing testing of patients with out of specification instruments and /or expired supplies and or uncertified heath care professional.

4. A method according to the preceding claims wherein the upload data are stored in a central database.

5. A method according to claim 4 wherein the upload data are to be accessed by the host computer (14) and /or by the a third party user.

6. A method according to the preceding claims wherein the detection via host computer (14) of the connection of each instrument (10,86) to the data communication network is performed by a network monitor function.

7. A method according to claim 6 wherein the network monitor function can be initiated when the host computer (14) is booted or when data review and instrument setup functions are started or a user start the network monitor function executable.

8. A method according to claim 6 wherein the network monitor function once initiated runs continuously on the host computer (14).

9. A method according to the preceding claims wherein the upload and the download data transfer is controlled by a communication manager.

10. A method according to the preceding claims wherein the operational review allows to view and edit information.

11. A method according to claim 10 wherein edit information consists in entering new data.

12. A method according to claim 11 wherein entering new data comprise entering notifications and warning based on the review of data uploaded from the instruments (10,86).

13. A method according to the preceding claims comprising displaying data on the display (26) of the hand-held instruments (10,86) including patient ID data, time and date of test data, analyte type data, numeric comments codes, and text descriptions of said numeric comment codes.

## Patentansprüche

1. Ein Verfahren zur Verwaltung von Daten für eine Vielzahl tragbarer Analyt-Testinstrumente (10, 86), die entnehmbar in entsprechenden Docking-Stationen (12, 70, 84) aufgenommen und mit einem Datenkommunikationsnetzwerk verbunden werden; folgende Schritte umfassend:
Lesen von Kalibrierungsdaten, die mit einem Teststreifen (40) verknüpft sind, mit einem Strichcode-Lesegerät,
Einführen des Teststreifens (40) in eine Öffnung (36) eines ersten tragbaren Testinstruments der Vielzahl tragbarer Analyt-Testinstrumente (10, 86),
Auftragen einer Probe auf den Teststreifen (40),
Verarbeiten eines Analyt-Signals, das vom Teststreifen (40) empfangen wird, um Analyt-Daten zu erzeugen,
Verbinden des ersten tragbaren Testinstruments mit einer der Docking-Stationen (12, 70, 84),
Erfassen der Verbindung jedes Instruments (10, 86) mit dem Datenkommunikationsnetzwerk über einen Hostrechner (14),
Heraufladen von Daten, die von jedem Instrument (10, 86) empfangen werden, einschließlich der Analyt-Daten, auf den Hostrechner (14),
Verarbeiten der heraufgeladenen Daten auf dem Hostrechner zur Überprüfung durch den Bediener,
Herunterladen von Konfigurationsdaten vom Hostrechner (14) auf jedes Testinstrument (10, 86), wobei die heruntergeladenen Daten instrumentenspezifische Einrichtungs- und Steuerungsdaten umfassen.

2. Ein Verfahren gemäß Anspruch 1, worin jedes der Analyt-Testinstrumente (10, 86) eine Teststreifen-Öffnung (36) einschließt, die Teststreifen (40) zur Bestimmung der Menge von Analyt in einer von einem Patienten genommenen Probe aufnimmt.

3. Ein Verfahren gemäß den Ansprüchen 1 oder 2, das weiter ein Sicherheitssystem zur Verhinderung des Testens von Patienten mit nicht spezifikationsgemäßen Instrumenten und/oder abgelaufenen Mitteln und/oder nicht zertifiziertem medizinischen Personal umfasst.

4. Ein Verfahren gemäß den obigen Ansprüchen, worin die heraufgeladenen Daten in einer zentralen Datenbank gespeichert werden.

5. Ein Verfahren gemäß Anspruch 4, worin auf die heraufgeladenen Daten vom Hostrechner (14) und/oder einem Dritten zugegriffen werden kann.

6. Ein Verfahren gemäß den obigen Ansprüchen, worin die Erfassung der Verbindung jedes Instruments (10, 86) mit dem Datenkommunikationsnetzwerk über den Hostrechner (14) durch eine Netzwerk-Überwachungsfunktion durchgeführt wird.

7. Ein Verfahren gemäß Anspruch 6, worin die Netzwerk-überwachungsfunktion initiiert werden kann, wenn der Hostrechner (14) hochgefahren wird oder wenn Datenüberprüfungs- und Instrumenten-Konfigurations-Funktionen gestartet werden oder ein Benutzer die ausführbare Netzwerk-Überwachungsfunktion startet.

8. Ein Verfahren gemäß Anspruch 6, worin die Netzwerk-Überwachungsfunktion, sobald sie gestartet wurde, kontinuierlich auf dem Hostrechner (14) läuft.

9. Ein Verfahren gemäß den obigen Ansprüchen, worin die Herauflade- und Herunterlade-Datenübertragung von einem Kommunikationsverwalter gesteuert wird.

10. Ein Verfahren gemäß den obigen Ansprüchen, worin die Bediener-Überprüfung es ermöglicht, Informationen zu überprüfen und zu editieren.

11. Ein Verfahren gemäß Anspruch 10, worin die Editierung von Informationen in der Eingabe neuer Daten besteht.

12. Ein Verfahren gemäß Anspruch 11, worin die Eingabe neuer Daten die Eingabe von Benachrichtigungen und Warnung auf der Grundlage der Überprüfung von Daten umfasst, die von den Instrumenten (10, 86) heraufgeladen wurden.

13. Ein Verfahren gemäß den obigen Ansprüchen, das Folgendes umfasst: die Anzeige von Daten auf dem Display (26) der tragbaren Instrumente (10, 86), einschließlich von Patienten-Identifikationsdaten, Zeit und Datum der Testdaten, Daten zum Analyt-Typ, numerischen Kommentar-Codes und Textbeschreibungen der numerischen Kommentar-Codes.

## Revendications

1. Procédé de gestion de données pour une pluralité d'instruments de test d'analyte portatifs (10, 86) reçus de manière amovible dans des stations d'accueil respectives (12, 70, 84) et connectés à un réseau de communication de données, comprenant les étapes consistant à :
lire des données de calibrage associées à une bandelette de test (40) avec un lecteur de codes-barres ;
insérer la bandelette de test (40) dans un port (36) d'un premier instrument de test portatif de la pluralité d'instruments de test d'analyte portatifs (10, 86) ;
appliquer un échantillon à la bandelette de test (40) ,
traiter un signal d'analyte reçu de la bandelette de test (40) afin de générer des données d'analyte ;
connecter le premier instrument de test portatif à une des stations d'accueil (12, 70, 84) ;
détecter via un ordinateur hôte (14) la connexion de chaque instrument (10, 86) au réseau de communication de données ;
téléverser des données reçues de chaque instrument (10, 86) vers l'ordinateur hôte (14) incluant les données d'analyte ;
traiter les données téléversées sur l'ordinateur hôte pour l'examen par un opérateur ;
télécharger des données de configuration depuis l'ordinateur hôte (14) vers chaque instrument de test (10, 86), les données téléchargées comprenant des données de réglage et de commande spécifiques à l'instrument.

2. Procédé selon la revendication 1, dans lequel chacun des instruments de test d'analyte (10, 86) inclut un port de bandelette de test (36), qui accepte des bandelettes de test (40) pour déterminer le niveau d'analyte dans un échantillon prélevé sur un patient.

3. Procédé selon la revendication 1 ou 2, comprenant en outre un système de sécurité pour empêcher le test de patients avec des instruments qui ne répondent pas aux spécifications et / ou des fournitures expirées et / ou un professionnel de la santé non diplômé.

4. Procédé selon les revendications précédentes, dans lequel les données de téléversement sont stockées dans une base de données centrale.

5. Procédé selon la revendication 4, dans lequel les données de téléversement doivent être accédées par l'ordinateur hôte (14) et / ou par un utilisateur tiers.

6. Procédé selon les revendications précédentes, dans lequel la détection via l'ordinateur hôte (14) de la connexion de chaque instrument (10, 86) au réseau de communication de données est effectuée par une fonction de moniteur de réseau.

7. Procédé selon la revendication 6, dans lequel la fonction de moniteur de réseau peut être initiée lorsque l'ordinateur hôte (14) est démarré ou lorsque des fonctions d'examen de données et de réglage d'instrument sont lancées ou qu'un utilisateur lance la fonction de moniteur de réseau exécutable.

8. Procédé selon la revendication 6, dans lequel la fonction de moniteur de réseau une fois initiée s'exécute en continu sur l'ordinateur hôte (14).

9. Procédé selon les revendications précédentes, dans lequel le transfert des données de téléversement et de téléchargement est contrôlé par un gestionnaire de communication.

10. Procédé selon les revendications précédentes, dans lequel l'examen opérationnel permet de visualiser et d'éditer des informations.

11. Procédé selon la revendication 10, dans lequel l'édition d'informations consiste à entrer de nouvelles données.

12. Procédé selon la revendication 11, dans lequel l'entrée de nouvelles données comprend l'entrée de notifications et d'avertissements sur la base de l'examen des données téléversées depuis les instruments (10, 86).

13. Procédé selon les revendications précédentes, comprenant l'affichage de données sur l'afficheur (26) des instruments portatifs (10, 86) incluant des données d'ID de patient, l'heure et la date des données de test, des données de type d'analyte, des codes de commentaires numériques, et des descriptions textuelles desdits codes de commentaires numériques.
